# DEMANDE DE BREVET EUROPEEN

(11) **EP 0 687 900 A1**
(43) Date de publication de la demande: **20.12.1995**
(21) Numéro de dépôt: 95201572.5
(22) Date de dépôt: 13.06.1995
(51) Int. Cl.: G01N 7/22, G01N 33/10

(54) **Procédé et installation de mesure des caractéristiques rhéologiques et fermentatives des pâtes à base de farine**

(30) Priorité: 16.06.1994 FR 9407609
(71) Demandeur: Vanneste, Jacques, B-9051 Gent (BE)
(72) Inventeur: Vanneste, Jacques, B-9051 Gent (BE)
(74) Mandataire: Donné, Eddy

(57) **Abrégé**

Procédé de mesure de l'évolution des caractéristiques rhéologiques et fermentatives des pâtes à base de farine à partir d'un pâton (1) de volume V de pâte, caractérisé par le fait que :
- on laisse fermenter ledit pâton (1) sous une pression initiale Po,
- on soumet ledit pâton (1) à une expansion forcée (2) et à un dégazage partiel par baisse de la pression P,
- on mesure l'évolution du volume V dudit pâton (1) pour contrôler son expansion,
- on comprime ledit pâton (1), partiellement dégazé, par un retour vers la pression initiale Po.

La demande concerne également une installation de mesure pour la mise en oeuvre du procédé.

## Description

La présente invention concerne un procédé et une installation de mesure de l'évolution des caractéristques rhéologiques et fermentatives des pâtes à base de farine, notamment ensemencées de levures ou additionnées d'agents levants chimiques, pour lesquelles on souhaite connaître par exemple, l'expansibilité, et/ou la résistance à l'expansibilité. Il peut s'agir, notamment, de pâtes à base de farine de céréales.

La mesure est réalisée par simulation, notamment en laboratoire, de diagrammes de panification. Elle permet, par exemple, de donner des indications sur la valeur boulangère des farines comme leur pouvoir de rétention gazeuse et de développement au four.

Bien que plus particulièrement développée pour le domaine agro-alimentaire et tout spécialement l'industrie meunière et boulangère, ce procédé et cette installation de mesure peuvent néanmoins s'appliquer à tous les domaines où l'on souhaite mesurer les caractéristiques rhéologiques d'un matériau mou et expansible, contenant notamment une phase gazeuse.

Jusqu'à présent, l'essai de panification réalisé par l'homme de l'art lui-même est le seul procédé qui donne une évalutation correcte de la qualité boulangère d'une farine de céréales panifiables.

Cependant, ces essais présentent des difficultés de reproductibilité car ils font intervenir un savoir-faire particulier. Ils présentent également le problème que pose l'interprétation, en grande partie subjective, de l'évolution de l'essai et du résultat final.

Pour ces raisons, des appareils de laboratoire ont été mis au point ayant pour but de prédire, d'une façon simple et reproductible, la qualité boulangère des farines.

Une première catégorie d'appareils mesurent les qualités rhéologiques des pâtes de farine panifiable, notamment leur extensibilité uni ou bi-axiale et leur résistance à la contrainte. Sur la base de ces paramètres, ils donnent principalement la force d'une farine.

Cependant, cette force n'est qu'un des aspects de la valeur boulangère d'une farine. Entre autres, vu que dans cette catégorie d'appareils, on utilise des pâtes non ensemencées de levure, la corrélation de la force avec, par exemple, le volume du pain obtenu dans des essais de panification est aléatoire.

Un autre inconvénient de ces essais d'extension est d'être destructif et de ne pas donner une évolution dans le temps des caractéristiques rhéologiques de la pâte.

Une seconde catégorie d'appareils opère sur des pâtes ensemencées de levure et donnent l'évolution de la résistance et/ou de la réaction de ces pâtes à une compression, constante ou intermittente, durant une partie ou la totalité de la durée de fermentation. L'interprétation des mesures donne principalement des indications sur la tenue ou la maturité des pâtes.

Toutefois, ces valeurs ne révèlent que partiellement la valeur boulangère d'une farine. En effet, ils ne mesurent pas l'évolution de l'extensibilité de la pâte qui est une des seules caractéristiques ayant une corrélation avec la valeur boulangère de la farine qu'elle contient comme, notamment le volume du pain.

La présente invention a pour but de remédier aux lacunes précédentes en proposant un procédé et un dispositif de mesure, entre autres, de l'évolution de l'expansibilité et/ou de la résistance à l'expansibilité d'une pâte de farine de céréales, notamment ensemencée de levures ou additionnée d'un agent levant chimique, et ainsi de prédire, par exemple, son pouvoir de développement au four lors de la panification.

Un autre but de la présente invention est de proposer un procédé et une installation de mesure simultanée de l'évolution de l'activité fermentative d'une pâte et de l'évolution de ses caractéristiques rhéologiques comme son expansibilité d'où est déduite son extensibilité.

Un autre but de la présente invention est de proposer un procédé et une installation de mesure opérant sur des pâtes qui peuvent être de volume constant, de consistance constante ou à hydratation constante après pétrissage.

Un autre but de la présente invention est de proposer un procédé et une installation de mesure qui peuvent simuler des diagrammes de panification depuis le début de la fermentation jusqu'aux premières minutes de la cuisson au four. Ceci par une série d'essais sur le même échantillon de pâte grâce à des opérations non destructives.

Un autre but de la présente invention est de proposer un procédé et une installation de mesure permettant une expansion de la pâte fermentante dans toutes les directions de l'espace.

Un autre but de la présente invention est de proposer un procédé et une installation de mesure permettant d'étudier l'effet d'additifs, dits régulateurs ou améliorants, ajoutés aux farines.

Un autre but de la présente invention est de proposer un procédé et une installation de mesure où la commande des opérations soit simple et efficace et ne nécessite pas l'intervention d'un homme de l'art.

Un autre but de la présente invention est de proposer un procédé et une installation de mesure qui peuvent fonctionner avec des volumes de fermentation prédéterminés, ou avec des durées de fermentation prédéterminées.

Un avantage de la présente invention réside dans le fait qu'elle est applicable aussi bien à un laboratoire de recherche qu'à un laboratoire d'essais ou de contrôle qualité industrielle.

D'autres buts et avantages de la présente invention apparaîtront au cours de la description qui va suivre qui n'est donnée qu'à titre indicatif et qui n'a pas pour but de la limiter.

A cet effet, selon l'invention, le procédé de mesure de l'évolution des caractéristiques rhéologiques et fermentatives des pâtes à base de farine à partir d'un pâton de volume V de pâte, est caractérisé par le fait que:
- on laisse fermenter ledit pâton sous une pression initiale Po,
- on soumet ledit pâton à une expansion forcée et à un dégazage partiel par baisse de la pression P,
- on mesure l'évolution du volume V dudit pâton pour contrôler son expansion,
- on comprime ledit pâton, partiellement dégazé, par un retour vers la pression initiale Po.

Selon une mise en oeuvre du procédé conforme à l'invention, l'on pétrit tout d'abord la pâte et on forme le pâton de volume V en donnant à la pâte une forme sensiblement sphérique ayant un état de surface pratiquement constant.

Selon une autre caractéristique du procédé de l'invention, on immerge ledit pâton dans un liquide hydrophobe.

La présente invention a également pour objet une installation de mesure pour la mise en oeuvre du procédé de mesure de l'évolution des caractéristiques rhéologiques et fermentatives des pâtes à base de farine à partir d'un pâton de volume V de pâte, caractérisée par le fait qu'elle comporte :
- des moyens pour contenir et laisser fermenter ledit pâton,
- des moyens pour abaisser la pression P, à partir d'une pression initiale Po, apte à soumettre ledit pâton à une expansion forcée et à un dégazage partiel,
- des moyens pour mesurer l'évolution du volume V dudit pâton,
- des moyens pour faire retourner la pression P vers son niveau initial Po, apte à comprimer ledit pâton, partiellement dégazé.

Selon une caractéristique additionnelle de l'installation de mesure conforme à l'invention, elle comporte des moyens pour former ledit pâton.

L'invention sera bien comprise à la lecture de la description suivante accompagnée des dessins en annexe parmi lesquels :
- les figures 1a à 1e représentent l'évolution du volume V du pâton pendant les différentes étapes du procédé de mesure,
- la figure 2 montre une courbe présentant les résultats d'un relevé de plusieurs mesures successives effectuées sur le même pâton,
- la figure 3 montre une vue de coupe qui illustre un premier mode de réalisation de l'installation de mesure,
- la figure 4 montre une vue de coupe qui détaille la structure d'un élément présenté dans la figure 3 précédente,
- la figure 5 est une vue de coupe d'une pièce coopérant avec l'élément présenté dans la figure 4,
- la figure 6 est une vue de dessus de l'élément présenté dans la figure 4,
- la figure 7 est une vue de dessus partielle de la pièce présentée dans la figure 5,
- la figure 8 est une vue de coupe qui illustre un second mode de réalisation de l'installation de mesure,
- la figure 9 présente, en coupe, une variante de réalisation, d'après la figure 8 précédente, des moyens de mesure de l'évolution du volume V du pâton,
- la figure 10 est une vue de dessus d'après la figure 9,
- la figure 11 détaille, en vue de coupe, un mode de réalisation de l'installation de mesure présentant les moyens pour former le pâton.

La présente invention a pour objet un procédé de mesure de l'évolution des caractéristiques rhéologiques et fermentatives des pâtes à base de farine, notamment à base de farine de céréales et ensemencées de levures ou additionnées d'agents levants chimiques.

Elle trouvera notamment son application dans des secteurs de l'activité agro-alimentaire comme l'industrie meunière et boulangère où l'on souhaite connaître l'évolution des caractéristiques rhéologiques et fermentatives des pâtes à base de farine de céréales panifiables afin de déterminer, entre autres, la qualité d'une préparation panaire par une simulation significative et reproductible des étapes des diagrammes de panification connues de l'homme du métier.

La présente invention s'applique ainsi par exemple à l'évaluation de la valeur boulangère des farines contenues dans ces préparations panaires.

L'application de l'invention à l'industrie boulangère n'est toutefois pas limitative et peut s'étendre à tous les domaines de l'activité économique où l'on souhaite mesurer les caractéristiques d'un matériau mou et expansible, contenant notamment une phase gazeuse.

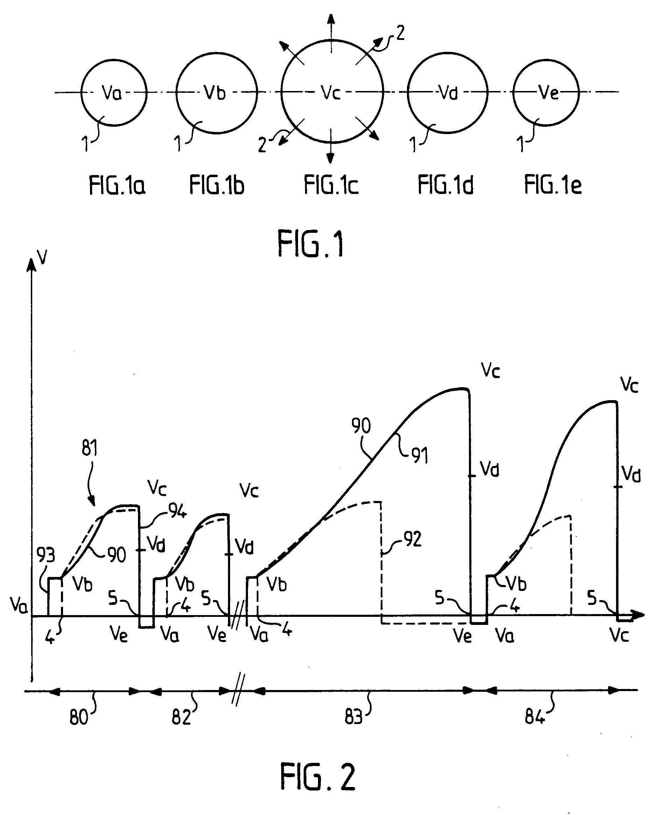

La figure 1 illustre les différentes étapes du procédé de mesure. Ce procédé travaille à partir d'un pâton 1 de volume V de pâte.

Comme représenté à la figure 1a, on laisse fermenter le pâton sous une pression initiale Po. Il peut s'agir, par exemple, de la pression atmosphérique.

A partir d'un volume de départ Va, le pâton atteint, sous l'action de la fermentation, un volume Vb comme représenté à la figure 1b.

On soumet alors le pâton 1 à une expansion forcée 2 par baisse de la pression P. Sous dépression, le pâton, qui contient des gaz, évolue jusqu'à un volume Vc comme représenté à la figure 1c.

Suivant le pouvoir de rétention gazeuse de la pâte dont est formé le pâton, les gaz commencent à s'en échapper et le pâton est soumis à un dégazage partiel jusqu'à un volume Vd comme représenté à la figure 1d.

On mesure l'évolution de son volume V pour contrôler son expansion.

Enfin, on comprime le pâton 1 partiellement dégazé par un retour vers la pression initiale Po. Comme représenté à la figure 1e, le pâton peut revenir à un volume Ve, par exemple, sensiblement identique à son volume de départ Va.

Selon un mode de réalisation de la présente invention, on pétrit tout d'abord la pâte et on forme ensuite le pâton 1 de volume V en donnant à la pâte une forme sensiblement sphérique ayant un état de surface pratiquement constant.

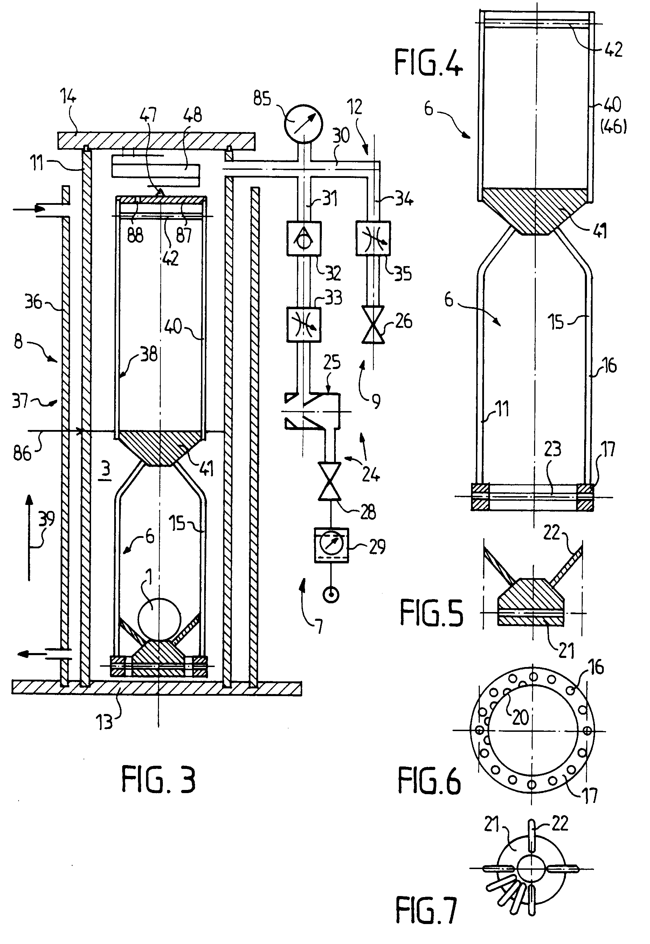

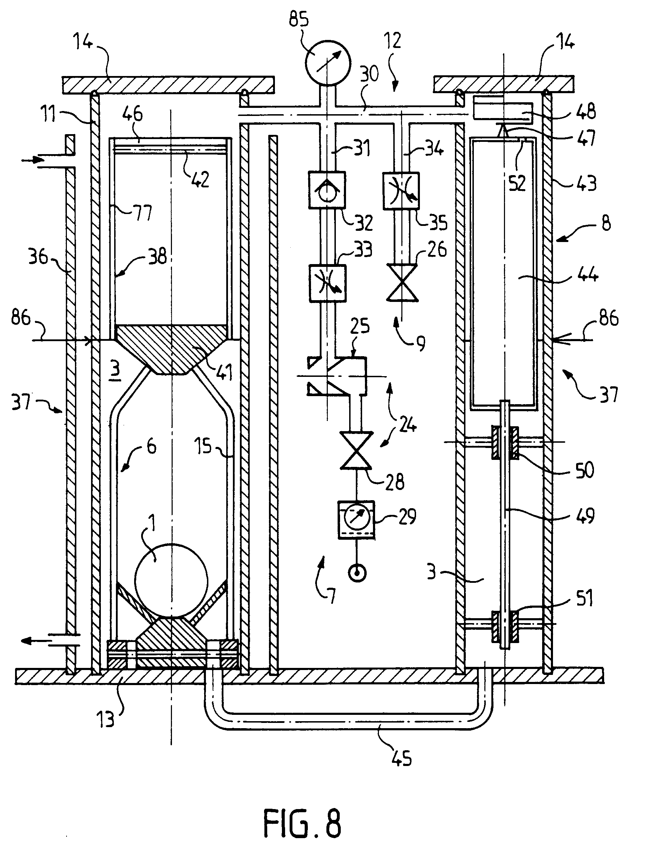

Avantageusement, selon l'invention, on immerge le pâton 1 dans un liquide 3 de préférence hydrophobe. Il peut s'agir, par exemple, d'huile de vaseline. Cette étape s'effectue, par exemple, préalablement à l'étape évoquée plus haut dans laquelle on laisse fermenter le pâton 1 sous une pression initiale Po. Le niveau du liquide après immersion du pâton à volume Va se trouvera, par exemple, au repère 86 sur les figures 3 et 8.

Comme développé plus amplement par la suite, le liquide hydrophobe 3 présente, entre autres, quatre avantages. Le premier est de permettre la fermentation à une température constante de la pâte s'y trouvant immergée, le second avantage est de participer à la mesure, le troisième avantage étant d'éviter la déshydratation superficielle du pâton, le quatrième avantage de réduire le coefficient de friction qui s'oppose sinon d'une façon non constante à l'expansion du pâton.

La mesure est réalisée, par exemple, en continu. Ainsi, on surveille l'évolution du volume V du pâton 1 lors de la fermentation sous pression Po.

Par surveillance, on entend une mesure du temps de fermentation nécessaire pour obtenir une augmentation donnée du volume V du pâton, sous pression Po, ou une mesure de l'agumentation du volume V du pâton pour une durée de fermentation donnée sous pression Po.

On effectue également au moins une mesure de l'évolution du volume V du pâton 1 lors de l'expansion forcée 2 et une mesure de l'évolution du volume V du pâton 1 lors du dégazage partiel et lors de la compression par retour vers la pression Po.

Cette série de mesures peut constituer un essai. Comme montré à la figure 2, où l'axe des abscisses représente le temps et l'axe des ordonnées représente l'évolution du volume V du pâton, l'essai repéré 80 correspond à une première partie 81, notamment une droite 93 dont la hauteur figure le volume de fermentation, qui dans l'exemple présent est une constante. Le pâton part d'un volume Va pour atteindre un volume Vb. Le temps nécessaire pour obtenir le volume Vb est chronométré.

Ensuite, une courbe 90 donne l'évolution du volume jusqu'au volume maximal Vc à une date T₁ repéré en 5, la date origine To étant repérée en 4.

Enfin, une droite 94 figure la baisse de volume suite au dégazage partiel jusqu'au volume Vd et suite au retour à Po jusqu'au volume Ve.

Le procédé n'étant pas destructif, on peut répéter plusieurs essais 82, 83, 84 pour déterminer ainsi l'évolution des caractéristiques rhéologiques et fermentatives du pâton et obtenir des indications sur les qualités de la farine à partir de laquelle elle est composée.

Comme représenté à la figure 2, on obtient la courbe 90 sur laquelle on relève lors de l'expansion forcée 2 le volume Vc appelé volume d'expansion maximum, qui représente un indice de l'extensibilité et du pouvoir de rétention gazeuse de la pâte. On relève aussi les temps 5 nécessaires pour atteindre ce volume d'expansion maximum et obtenir ainsi des indications sur la résistance de la pâte à son expansion.

L'évolution de la vitesse de fermentation, durant les expériences représentées à la figure 2, est déduite du chronométrage des temps nécessaires pour obtenir les volumes Vb qui sont constants dans cet exemple.

L'évolution, lors des différents essais, des grandeurs mesurées permet entre autres d'évaluer la qualité boulangère de la pâte et de la farine qu'elle contient. Le nombre d'essais dépend du diagramme de panification que l'on souhaite simuler.

On peut ainsi comparer la valeur boulangère de deux farines différentes. La première, dont l'évolution de l'expansion est repérée en 91 par le trait continu, est dite "forte". La seconde, dont l'évolution est repérée en 92 par le trait en pointillé est dite "faible". D'après les résultats de la mesure, on peut prédire que cette dernière donnera entre autres des pains moins volumineux que la première.

Selon l'invention, lors de l'expansion forcée 2, on extrait pratiquement le volume de gaz des produits obtenus durant la fermentation comme le gaz carbonique ainsi qu'une partie de l'alcool. Ce dégazage ne doit cependant pas être complet.

L'invention a également pour objet une installation de mesure pour la mise en oeuvre du procédé de mesure. On travaille à partir d'un pâton 1 de volume V de pâte.

Comme représentée aux figures 3 et 8, elle comporte des moyens 6 pour contenir et laisser fermenter le pâton 1. Elle comporte également des moyens 7 pour abaisser la pression P, à partir d'une pression initiale Po. Ces moyens 7 pour abaisser la pression P sont aptes à soumettre le pâton 1 à une expansion forcée 2 et à un dégazage partiel.

L'installation de mesure comporte également des moyens 8 pour mesurer l'évolution du volume V du pâton 1 et des moyens 9 pour faire retourner la pression P vers son niveau initial Po, apte à comprimer le pâton 1 partiellement dégazé.

Selon une caractéristique additionnelle de l'invention, l'installation de mesure comporte également des moyens 10 pour former le pâton 1. Ces moyens permettent de travailler avec un pâton reproductible en lui donnant un poids et/ou un volume pratiquement constant, une forme géométrique déterminée ainsi qu'un état de surface de qualité pratiquement constante, ceci à partir de pâtes ayant des consistances usuelles en panification.

Les moyens 6 pour contenir et laisser fermenter le pâton 1 comportent, par exemple, un récipient 11, muni de moyens 12 d'équilibrage de la pression par rapport à la pression extérieure. Ce récipient 11 se présente, par exemple, sous une forme cylindrique. Il peut être réalisé dans une matière transparente comme celle connue sous le nom de plexiglas. Il est fixé d'une façon étanche sur un socle 13. Un couvercle 14, apte à assurer une fermeture hermétique, est assujetti à l'extrémité supérieure du récipient 11.

Le récipient 11 présente, en outre, par exemple, une jacquette thermostatique constituée d'une enveloppe cylindrique 36 coaxiale au récipient 11. Cette dernière est de préférence en matière transparente comme par exemple celle connue sous le nom de plexiglas. Elle est fixée sur le socle 13 de manière étanche. Une pompe fait circuler de l'eau qui passe par un réservoir chauffé par une résistance commandée par un thermostat non représenté sur le dessin.

Toujours suivant les figures 3 et 8, les moyens 6 pour contenir et laisser fermenter le pâton 1 comporte également un liquide hydrophobe 3 contenu dans le récipient 11.

Suivant les figures 4 à 7, les moyens 6 pour contenir et laisser fermenter le pâton 1 comporte, en outre, une cage 15, incluse dans le récipient 11. Le pâton 1 est placé à l'intérieur de cette cage 15 qui présente une forme et une structure aptes à laisser évoluer le volume V de ce pâton 1 dans toutes les directions de l'espace et ceci dans des limites qui empêchent une déformation ovale excessive. Aussi, la longueur de l'espace cylindrique, délimité par la cage 15, permet au pâton de monter et descendre selon sa densité qui peut ainsi être observée.

Suivant le mode de réalisation représenté, la cage 15 est constituée de tiges de section ronde 16, fixées dans une couronne 17, qui délimitent ainsi sur une partie de leur longueur un espace cylindrique, pour ensuite se rassembler dans une pièce 41 de forme cylindro-troncônique. Dans la face intérieure de la couronne 17, sont ménagées des encoches 20.

La cage 15 présente également un dispositif de fermeture amovible 21 de forme cylindro-troncônique dans la partie cônique de laquelle sont fixées des broches 22 en même nombre que les tiges 16 de la cage 15.

Les broches 22 présentent des pointes pouvant passer par les encoches 20. Le dispositif de fermeture amovible 21 est ainsi apte à être introduit dans la cage 15 pour l'obturer par rotation afin de faire coïncider les pointes des broches 22 avec l'axe des tiges 16. Pour bloquer le dispositif de fermeture amovible 21 en translation verticale, la cage 15 comporte, en outre, une broche 23 coopérant avec un alésage percé radialement dans ce dispositif de fermeture amovible 21. Cette broche 23 permet également le centrage du dispositif de fermeture amovible 21 dans la cage 15. La cage 15 et le dispositif de fermeture 21 peuvent être garnis de matière connue sous le nom de téflon.

Les moyens 7 pour abaisser la pression P et les moyens 9 pour faire retourner la pression P à son niveau initial Po sont constitués de moyens 24 de variation de la pression. Pour résister à ces variations de pression, le récipient 11 est d'ailleurs prévu apte à résister en ce sens.

Les moyens 24 pour faire varier la pression sont constitués d'une pompe à vide 25 et d'une vanne de retour 26 en pression.

La pompe à vide 25 est constituée, par exemple, d'une pompe de type venturi multi-étages à air comprimé alimentée par une vanne 28 contrôlée par un régulateur de pression 29.

D'autre part, le récipient 11 présente un conduit 30 relié à la pompe 25 par un embranchement 31. Entre cette pompe 25 et l'embranchement 31, on trouve un clapet anti-retour 32 et un étrangleur réglable 33. Un manomètre 85 est assujetti au conduit 30.

La vanne de retour en pression 26 est complétée par un étrangleur réglable 35.

Le récipient 11 peut être ainsi mis sous dépression en mettant en route la pompe 25 ceci en ouvrant la vanne 28. A l'arrêt de la pompe 25, la dépression dans l'appareil est maintenue par la fermeture du clapet anti-retour 32. La vitesse à laquelle l'appareil est mis sous dépression ou remis sous pression est réglée par les étrangleurs réglables 33, 35.

Les moyens 8 de mesure de l'évolution du volume V du pâton 1 sont constitués de moyens 37 pour convertir l'évolution du volume V en un signal mesurable, variable unidirectionnellement, et de moyens 38 pour amplifier ledit signal.

Suivant un premier mode de réalisation représenté à la figure 3, les moyens 37 pour convertir l'évolution du volume V en un signal mesurable, variable unidirectionnellement, sont constitués, d'une part par la cage 15, prévue flottante. Elle transmet ainsi la poussée 39 qu'elle subit par l'action de la variation de volume du pâton 1 qu'elle contient.

D'autre part, les moyens 38 pour amplifier ledit signal sont constitués par un flotteur 40, assujetti au-dessus de la cage 15. Il permet d'amplifier la poussée 39 par amplification des variations du niveau du liquide déplacé, notamment, à son voisinage.

Le flotteur 40 présente une forme, par exemple, cylindrique. Il est relié à la cage 15 par l'intermédiaire de la pièce de liaison 41 de forme cylindro-troncônique prévue elle aussi flottante.

Le flotteur 40 comprend, en outre, par exemple, une poignée 42 placée à son extrémité supérieure. Celle-ci permet de manipuler l'ensemble flotteur 40 et cage 15 afin de les introduire et/ou de les sortir du récipient 11. Il peut être fermé, suivant la figure 3, par un couvercle amovible 87, muni en son centre d'une pointe à bille 47, percé d'un trou 88 pour l'égalisation des pressions.

Selon un second mode de réalisation représenté à la figure 8, les moyens 37 pour convertir l'évolution du volume V en un signal mesurable, variable unidirectionnellement, sont constitués d'un second récipient 43 communiquant avec le premier récipient 11 et d'un flotteur 44 placé dans le second récipient 43 qui est, par exemple, cylindrique.

Le récipient 11 et le récipient 43 sont reliés au niveau du socle 13 par une conduite 45 qui assure la circulation du liquide, par exemple, hydrophobe 3 suivant le principe des vases communicants. Ils sont également reliés au niveau supérieur par le conduit 30 qui assure ainsi un même niveau de pression dans les deux récipients.

Le flotteur 44 est apte à produire une poussée variable avec le niveau du liquide hydrophobe 3.

A la figure 8, on remarque qu'une tige guide 49, maintient verticale la position du flotteur 44. Des coussinets 50-51, fixés au récipient 43 permettent la translation verticale de la tige guide 49. Le flotteur 44 est également muni, par exemple, d'un couvercle percé d'un trou 52 afin d'égaliser la pression intérieure avec la pression environnante.

D'autre part, les moyens 38 pour amplifier ce signal sont constitués par un élément volumique 46. Sa forme est complémentaire au récipient 11 et peut être, par exemple, cylindrique. Il est placé au-dessus de la cage 15 et laisse un intervalle de faible épaisseur entre sa surface extérieure 77 et la surface intérieure 78 du récipient 11. Ceci a pour but d'amplifier les variations du niveau du liquide hydrophobe 3. Le poids spécifique de l'ensemble, élément volumique 46 - cage 15, sera dans le cas présent nettement supérieur au poids spécifique du liquide hydrophobe 2, et repose sur le socle 13.

D'autre part, cet intervalle permet le passage du gaz qui s'échappe du pâton 1 lors du dégazage.

L'utilisation d'un second récipient 43 permet de reporter la mesure du niveau du liquide hydrophobe dans un milieu non perturbé par les dégagements gazeux du pâton 1 ou par la présence éventuelle de mousse.

D'après les figures 3 et 8, l'installation de mesure comporte, en outre, par exemple, une pointe à bille 47 placée au sommet des flotteurs 40, 44. Cette pointe à bille 47 coopère avec une jauge de contrainte 48 contre laquelle elle vient en appui.

Suivant des moyens connus de l'homme de l'art et non représentés sur ces figures, l'action de la pointe à bille sur la jauge de contrainte est convertie en un signal électrique dont les variations sont reportées par exemple, sur table traçante.

Suivant un mode de réalisation particulier représenté à la figure 6, l'installation de mesure et plus particulièrement le second récipient 43 comporte un chariot mobile 53, guidé en translation le long de la paroi extérieure verticale 79 de ce second récipient 43. Un premier aimant 54, placé dans le flotteur 44 coopère avec un second aimant placé dans le chariot mobile 53.

Ce chariot mobile 53 est muni d'une plume 56, apte à transcrire sur un enregistreur les variations de niveaux du flotteur 44. Pour cela, ce dernier est traversé axialement d'un conduit 57 à-travers lequel passe une tige 58 fixée au socle 13 et à l'extrémité supérieure 14 du second récipient 43. Des roulettes à gorge 59-60 coopèrent avec la tige 58 pour permettre une translation sans-à-coups du flotteur 44 malgré la force d'attraction des aimants 54-55.

D'autre part, pour faciliter cette translation sans-à-coups, le chariot mobile 53 comporte une tige filetée 61 sur laquelle est fixé le second aimant 55. La distance entre les aimants 54-55 est donc réglable. De plus, le chariot mobile 53 est guidé en translation par un et/ou des rails 62 coopérant avec des roulettes 63 montées sur le chariot mobile 53.

D'autre part, l'aimant 54 est serti d'une façon étanche dans la paroi du flotteur 44. De plus, un orifice 64 est prévu à l'extrémité supérieure de ce flotteur pour l'égalisation de la pression.

Les parties constituantes et celles se trouvant dans le récipient 43 seront construites de préférence en matières amagnétiques.

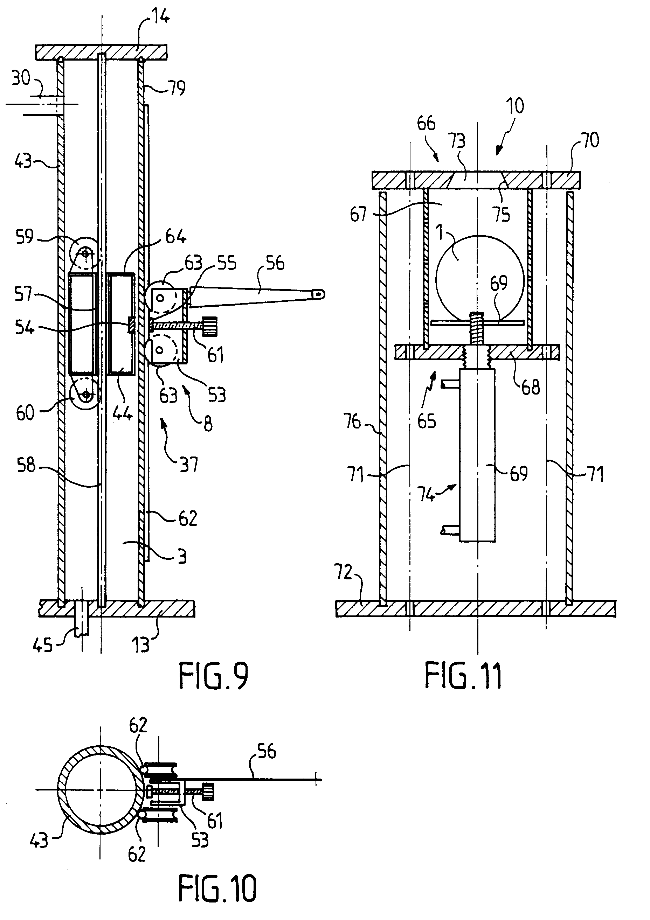

Comme représentés à la figure 11, les moyens 10 pour former le pâton 1 sont constitués de moyens 65 pour comprimer la pâte et de moyens 66 pour produire un pâton de volume V ayant une forme sensiblement sphérique ayant un état de surface pratiquement constant.

Les moyens 65 pour comprimer la pâte sont constitués d'une cavité 67, apte à recevoir la pâte. Cette cavité 67, par exemple de forme cylindrique, est munie de fines perforations sur une partie de sa surface pour permettre l'évacuation de l'air. Sa paroi est, par exemple, en matière transparente comme celle connue sous le nom de plexiglas et repose dans une rainure aménagée dans un socle 68.

Les moyens 65 pour comprimer la pâte sont également constitués d'un piston 69 coulissant à l'intérieur de la cavité 67. Ce piston 69 est actionné, par exemple, par un vérin pneumatique à course constante. La position basse du piston 69 est réglable de manière à avoir une cavité 67 de volume modulable. Les mouvements de ce piston sont commandés dans les deux sens par une vanne bidirectionnelle. Un jeu suffisant pour que l'air puisse s'échapper est prévu entre le piston 69 et la paroi de la cavité 67.

Les moyens 65 pour comprimer la pâte sont également constitués d'un couvercle 70. Quatre tiges filetées 71 sont fixées dans une plaque de support 72 de manière à soutenir le socle 68 et à fixer le couvercle 70.

Les moyens 66 pour produire un pâton de volume V ayant une forme sensiblement sphérique sont constitués d'un orifice 73 ménagé dans le couvercle 70. Lors de la compression de la pâte, cet orifice 73 est bouché hermétiquement par des moyens connus de l'homme de l'art. Pour permettre la formation du pâton 1, il est débouché.

Cet orifice 73 constitue, par exemple, une filière d'extrusion de dimension adaptée par rapport à la pâte, à la cavité 67 et au pâton que l'on souhaite obtenir.

Par exemple, en prenant comme référence le diamètre du pâton 1 sphérique que l'on souhaite obtenir, l'orifice 73 présente un diamètre de 0,6 et la cavité 67 un diamètre de 1,3 pour un diamètre de pâton égal à 1.

De plus, le volume de pâte introduit dans la chambre de compression sera toujours nettement supérieur au volume du pâton que l'on désire extruder.

Les moyens 66 pour produire un pâton de volume V ayant une forme sensiblement sphérique sont constitués également de moyens 74 de contrôle de la vitesse de la course du piston 69.

De plus, pour faciliter la formation d'un pâton 1 sensiblement sphérique, l'orifice 73 ménagé dans le couvercle 70 présente une paroi 75 d'inclinaison convergente par rapport à l'extérieur de la cavité 67. Par exemple, l'inclinaison de la paroi 75 de l'orifice 73 est de 60 degrés par rapport à l'horizontale. Cette paroi 75 peut être garnie d'une matière connue sous le nom de téflon.

L'installation de mesure conforme à l'invention, présente en outre, par exemple, à l'extérieur de l'orifice 73 des moyens pour découper le pâton 1 une fois extrudé tel qu'un fil fin en forme de boucle de préférence réalisé à partir d'une matière connue sous le nom de nylon.

D'autre part, une enceinte 76, de forme par exemple cylindrique, réalisée de préférence dans une matière connue sous le nom de plexiglas, contient la cavité 67. Cette enceinte 76 est recouverte par le couvercle 70. Une résistance électrique commandée par un thermostat non figurée sur le dessin, maintient l'enceinte 76 à une température constante.

Pour permettre une meilleure compréhension de la présente invention, on décrit ci-après un exemple de mode d'emploi de l'installation de mesure pour la mise en oeuvre du procédé de mesure.

Une expérience de mesure de l'évolution des caractéristiques rhéologiques et fermentatives d'une pâte se fait par une série d'essais qui comprennent chacun un cycle de trois phases.

Pour que les diverses expériences soient comparables en tous points, le poids et/ou le volume des pâtes mis en oeuvre doit être constant ainsi que les autres conditions, comme les réglages de mise sous vide et de mise sous pression atmosphérique, les températures et temps ou volumes de fermentation.

On pétrit une pâte, par exemple, de farine, de froment, d'eau, de sel et de levure et ceci dans des conditions déterminées. Après cela, on utilise les moyens 10 pour former la pâte.

Initialement, le piston 69 se trouve dans une position inférieure dans la cavité 67 à l'intérieur de laquelle on place une quantité suffisante de pâte. Avant de déposer la pâte sur le piston, on l'enduit d'huile.

Une fois la pâte en place à l'intérieur de la cavité 67, on fixe le couvercle 70 et on obture l'orifice 73 par exemple grâce à un poids présentant une surface anti-adhésive à son extrémité inférieure. On met alors le piston 69 en mouvement ascendant à la suite de quoi la pâte est comprimée et les poches d'air qu'elle peut contenir expulsées. La durée et le degré de compression sont des constantes d'une expérience à l'autre.

Ensuite, on enlève le poids et sous la pression du piston 69, on extrude la pâte par l'orifice 73.

Entre la compression et l'extrusion, on peut intercaler une période de relaxation de la pâte. Pour cela, le piston est mis en mouvement descendant et regagne sa position inférieure. Après un temps de relaxation donné, on exécute l'opération d'extrusion en mettant le piston 69 en mouvement ascendant après avoir ôté le poids qui bouche l'orifice 73.

La vitesse d'extrusion pour un volume donné de pâte est fonction de sa consistance.

Grâce aux caractéristiques visco-élastiques de la pâte, on obtient ainsi un pâton 1 ayant une forme géométrique déterminée, de préférence approximativement sphérique, d'un volume Va déterminé. L'état de la surface du pâton est d'une qualité aussi constante que possible. D'autre part, on évite l'inclusion de poches d'air, étant entendu que la pâte contient nécessairement de l'air finement dispersé.

Une fois formé, le pâton 1 est placé dans la cage 15 qui est engagée à l'intérieur du récipient 11 rempli d'un liquide hydrophobe 3.

Le couvercle 14 du récipient cylindrique 11 est fermé et la vanne 28 est ouverte. C'est le début de la première phase de l'essai. Elle consiste notamment en la fermentation sous pression atmosphérique jusqu'à obtention d'une augmentation de volume désiré, appelé volume de fermentation Vb.

Cette augmentation de volume se produit par la formation de gaz de fermentation à l'intérieur de la pâte et elle est déterminée de préférence de telle façon que pour toutes les pâtes, quel que soit leur pouvoir de rétention gazeuse, le gaz ne s'en échappera pratiquement pas encore.

Quand le volume de fermentation prédéterminé est atteint, la seconde phase de l'essai peut commencer. On ferme la vanne 26 et on met en route la pompe à vide 25. La dépression dans l'appareil s'établit de façon constante et progressive et se transmet au pâton par l'intermédiaire du liquide dans lequel il est immergé. Le gaz contenu dans le pâton va se dilater à la suite de quoi le pâton augmente en volume tout en opposant une résistance à son expansion.

A partir d'une certaine dépression, différentes selon les qualités rhéologiques de la pâte mise en oeuvre, le gaz commence par s'en échapper ce qui n'empêche pas le pâton 1 d'augmenter encore de volume jusqu'au moment où il atteint son maximum d'expansibilité repérée par son extension maximale Vc.

La dépression augmentant, le pâton 1 est dégazé. Ce dégazage est partiel et le pâton retombe à un volume transitoire Vd. La dépression maximum prédéterminée atteinte, on arrête la pompe à vide 25 en fermant la vanne 28, le clapet anti-retour 32 se ferme et la dépression dans l'appareil est maintenue durant un temps prédéterminé.

La troisième phase de l'essai peut commencer, notamment la mise sous pression atmosphérique, ceci en ouvrant la vanne de retour en pression 26. La pâte est alors comprimée à un volume Ve approchant de son volume initial Va. Ensuite peut débuter la première phase de l'essai suivant.

Ces essais sont réitérés autant de fois que désiré en fonction du diagramme de panification que l'on souhaite simuler.

La présente invention n'est pas limitée à la seule forme de réalisation qui a été décrite ci-dessus mais elle en embrasse toutes les variantes de réalisation.

## Revendications

1. Procédé de mesure de l'évolution des caractéristiques rhéologiques et fermentatives des pâtes à base de farine à partir d'un pâton (1) de volume V de pâte, caractérisé par le fait que :
- on laisse fermenter ledit pâton (1) sous une pression initiale Po,
- on soumet ledit pâton (1) à une expansion forcée (2) et à un dégazage partiel par baisse de la pression P,
- on mesure l'évolution du volume V dudit pâton (1) pour contrôler son expansion,
- on comprime ledit pâton (1), partiellement dégazé, par un retour vers la pression initiale Po.

2. Procédé de mesure selon la revendication 1, caractérisé par le fait que l'on pétrit tout d'abord la pâte et on forme le pâton (1) de volume V en donnant à la pâte une forme sensiblement sphérique ayant un état de surface pratiquement constant.

3. Procédé de mesure selon la revendication 1, caractérisé par le fait que l'on immerge ledit pâton (1) dans un liquide (3) hydrophobe.

4. Procédé de mesure selon la revendication 1, caractérisé par le fait que l'on surveille l'évolution du volume V dudit pâton (1), lors de la fermentation sous pression Po, et l'on effectue au moins:
- une mesure de l'évolution du volume V du pâton (1) lors de l'expansion forcée (2),
- une mesure de l'évolution du volume V du pâton (1) lors du dégazage partiel et lors de la compression par un retour vers la presssion Po.

5. Procédé de mesure selon la revendication 1, caractérisé par le fait qu'on extrait, lors de l'expansion forcée (2), des produits de la fermentation, comme l'alcool et le gaz carbonique.

6. Installation de mesure pour la mise en oeuvre du procédé de mesure de l'évolution des caractéristiques rhéologiques et fermentatives des pâtes à base de farine, à partir d'un pâton (1) de volume V de pâte selon la revendication 1, caractérisée par le fait qu'elle comporte :
- des moyens (6) pour contenir et laisser fermenter ledit pâton (1),
- des moyens (7) pour abaisser la pression P, à partir d'une pression initiale Po, aptes à soumettre ledit pâton (1) à une expansion forcée (2) et à un dégazage partiel,
- des moyens (8) pour mesurer l'évolution du volume V dudit pâton (1),
- des moyens (9) pour faire retourner la pression P vers son niveau initial Po, apte à comprimer ledit pâton (1) partiellement dégazé.

7. Installation de mesure suivant la revendication 6, caractérisée par le fait qu'elle comporte des moyens (10) pour former ledit pâton (1).

8. Installation de mesure selon la revendication 6, caractérisée par le fait que les moyens (6) pour contenir et laisser fermenter ledit pâton (1) comportent un récipient (11) muni de moyens (12) d'équilibrage de la pression par rapport à la pression extérieure, et un liquide hydrophobe (3) contenu dans ledit récipient (11).

9. Installation de mesure selon la revendication 8, caractérisée par le fait que les moyens (6) pour contenir et laisser fermenter ledit pâton (1) comportent une cage (15) incluse dans ledit récipient (11) dans laquelle est placé ledit pâton (1), ladite cage (15) présentant une forme et une structure aptes à laisser évoluer le volume V dudit pâton (1) dans toutes les directions de l'espace.

10. Installation de mesure selon la revendication 8, caractérisée par le fait que les moyens (7) pour abaisser la pression et les moyens (9) pour faire retourner la pression P à son niveau initial Po sont constitués de moyens (24) de variation de la pression et par le récipient (11), apte à résister à des variations de pression.

11. Installation de mesure selon la revendication 10, caractérisée par le fait que les moyens (24) pour faire varier la pression sont constitués d'une pompe à vide (25) et d'une vanne de retour (26) en pression.

12. Installation de mesure selon la revendication 9, caractérisée par le fait que les moyens (8) de mesure de l'évolution du volume V du pâton (1) sont constitués de moyens (37) pour convertir l'évolution du volume Y du pâton (1) en un signal mesurable, variable unidirectionnellement, et de moyens (38) pour amplifier ledit signal.

13. Installation de mesure selon la revendication 12, caractérisée par le fait que les moyens (37) pour convertir l'évolution du volume V en un signal mesurable, variable unidirectionnellement, sont constitués par la cage (15) prévue flottante, apte à transmettre la poussée (39) qu'elle subit par l'action de la variation de volume du pâton (1) qu'elle contient, et les moyens (38) pour amplifier ledit signal sont constitués par un flotteur (40) assujetti au-dessus de la cage, aptes à amplifier la poussée (39) par amplification des variations de niveau du liquide déplacé.

14. Installation de mesure selon la revendication 12, caractérisée par le fait que les moyens (37) pour convertir l'évolution du volume V en un signal mesurable, variable unidirectionnellement, sont constitués d'un second récipient (43), communiquant avec le premier récipient (11), et d'un flotteur (44), placé dans ledit second récipient (43), apte à produire une poussée variable avec le niveau du liquide hydrophobe (3), et les moyens (38) pour amplifier le signal sont constitués par un élément volumique (46), placé au-dessus de la cage (15), apte à laisser un intervalle de faible épaisseur entre la surface extérieure (77) dudit élément volumique (46) et la surface intérieure (78) dudit récipient (11) de manière à amplifier les variations de niveaux du liquide hydrophobe (3).

15. Installation de mesure selon les revendications 13 ou 14, caractérisée par le fait qu'elle comporte, en outre, une pointe à bille (47) placée au sommet du flotteur (40; 44), et d'une jauge de contrainte (48) contre laquelle ladite pointe à bille (47) vient en appui.

16. Installation de mesure selon la revendication 14, caractérisée par le fait qu'elle comporte en outre un chariot mobile (53), guidé en translation le long de la paroi verticale (79) du second récipent (43), d'un premier aimant (54), placé dans le flotteur (44) et d'un second aimant (55), placé dans ledit chariot mobile (53), coopérant avec ledit premier aimant (54).

17. Installation de mesure selon la revendication 7, caractérisée par le fait que les moyens (10) pour former le pâton (1) sont constitués de moyens (65) pour comprimer la pâte et de moyens (66) pour produire un pâton de volume V ayant une forme sensiblement sphérique ayant un état de surface pratiquement constant.

18. Installation de mesure selon la revendication 17, caractérisée par le fait que les moyens (65) pour comprimer la pâte sont constitués d'une cavité (67), apte à recevoir la pâte, d'un piston (69) coulissant à l'intérieur de ladite cavité (67), et d'un couvercle (70), apte à clore hermétiquement ladite cavité.

19. Installation de mesure selon la revendication 18, caractérisée par le fait que les moyens (66) pour produire un pâton de volume V ayant une forme sensiblement sphérique sont constitués d'un orifice (73), ménagé dans le couvercle (70), de dimensions adaptées par rapport à ladite pâte, à ladite cavité (67) et audit pâton à obtenir (1) et de moyens (74) de contrôle de la course du piston (69).

20. Installation de mesure selon la revendication 19, caractérisée par le fait que l'orifice (73) ménagé dans ledit couvercle (70) présente une paroi (75) d'inclinaison convergente par rapport à l'extérieur de la cavité (67).
